# EUROPÄISCHE PATENTANMELDUNG

(11) **EP 1 118 671 A1**
(43) Veröffentlichungstag der Anmeldung: **25.07.2001**
(21) Anmeldenummer: 00100256.7
(22) Anmeldetag: 18.01.2000
(51) Int. Cl.: C12P 5/02

(54) **Verfahren und Vorrichtung zum Erzeugen von methanhaltigem Biogas aus organischen Stoffen**

(71) Anmelder: Rebholz, Erich, Dr. med., 76571 Gaggenau (DE)
(72) Erfinder: Rebholz, Erich, Dr. med., 76571 Gaggenau (DE); Reithmayer, Johann, Dipl.-Ing.agr., 85072 Eichstätt (DE)

(57) **Zusammenfassung**

Zum Erzeugen von Biogas mit hohem Methananteil aus organischen Stoffen (1) wird eine dreistufiges Verfahren vorgeschlagen, das eine aerobe Fermentation (4), eine Verschwelung (7) und eine thermophile Methanfermentation (11) umfaßt.

## Beschreibung

Die Erfindung betrifft ein Verfahren und eine entsprechende Vorrichtung zum Erzeugen von methanhaltigem Biogas aus organischen Stoffen, insbesondere zum Erzeugen von Biogas mit hohem Methananteil. Bei solchen Verfahren werden die organischen Stoffe mittels lebender Mikroorganismen zersetzt und zu Methan umgewandelt.

Bei der Zersetzung organischer Substanzen entsteht Gas, das als alternative Energiequelle dienen kann. Dieses Gas wird aufgrund seiner Gewinnung häufig als Biogas bezeichnet. Ein wichtiger Bestandteil von Biogas ist Methan, das aus organischen bzw. pflanzlichen Substanzen oder deren Folgeprodukten durch Vermodern bzw. Zersetzen unter Luftabschluß entsteht. Es wird in großtechnischem Umfang durch Vergasen von Kohle oder bei petrochemischen Prozessen gewonnen und als Heizgas und für Verbrennungsantriebe sowie als Ausgangsmaterial für synthetische Produkte, beispielsweise Acetylen, Synthesegas, HCN und Chlorsubstitutionsprodukte verwendet.

Aufgrund der Bedeutung des Methan ist man bestrebt, bei der Erzeugung von Biogas ein hohen Methananteil zu erzielen. Nach dem Stand der Technik werden hierzu ein- oder zweistufige Fermentationsverfahren eingesetzt, bei denen aus organischen Stoffen mittels anaerober Fermentation Biogas mit einem Methananteil zwischen 40 % und 60 % gewonnen wird. Die übrigen Bestandteile des Biogases setzen sich dabei aus 25 % bis 55 % CO₂ sowie kleineren Anteilen von Stickstoff, Schwefelwasserstoff und anderen Komponenten zusammen.

Die bekannten Verfahren der anaeroben Fermentation zur Gewinnung von Methan aus organischen Stoffen sind somit hinsichtlich der Qualität des erzeugten Biogases und der Methanausbeute nicht voll befriedigend. Dabei ist insbesondere der hohe Schwefel- bzw. Schwefelwasserstoffanteil von etwa 2 % unerwünscht, da bereits Konzentrationen ab 0,1 % beim Betrieb von Motoren und dem damit verbundenen Einsatz von Katalysatoren störend sind.

Darüber hinaus weisen die bekannten Verfahren der anaeroben Fermentation weitere Nachteile auf. So liegt der Abbaugrad üblicherweise bei etwa 45 % der organischen Trockensubstanz und die Prozesse verlaufen relativ instabil, da die beteiligten Mikroorganismen empfindlich auf Milieuänderungen reagieren. Dies hat auch zur Folge, daß bei einer Unterbrechung des Verfahrens, beispielsweise zur Durchführung von Wartungen oder Reparaturen, und einer erneuten Inbetriebnahme ein wirtschaftliches Leistungsniveau erst nach ca. 12 bis 25 Wochen erreicht wird.

Ferner fallen bei den bekannten Verfahren ca. 30 % bis 70 % der eingesetzten Menge als nichtverwertbarer Reststoff an, der auf Deponien entsorgt werden muß. Auch sind die Umwandlungszeiten, d.h. der Zeitraum zwischen der Zuführung eines organischen Stoffes zu einem Prozeß bis zur Erzeugung von Biogas relativ lang und liegen im Bereich von 20 bis 30 Wochen.

Die bekannten Verfahren sind zwar hinsichtlich des Kohlendioxidhaushaltes umweltneutral, führen aber nicht zu einer Reduzierung der Kohlendioxidbelastung der Umwelt. Dabei ist auch zu berücksichtigen, daß das beim nicht gesteuerten Zersetzungsprozeß in freier Natur freigesetzte Methan etwa 30 mal stärker negativ belastend für den Treibhauseffekt ist als CO₂.

Der Erfindung liegt unter Berücksichtigung dieses Standes der Technik die Aufgabe zugrunde, ein Verfahren und eine entsprechende Vorrichtung zum Erzeugen von methanhaltigem Biogas aus organischen Stoffen, wobei die organischen Stoffe mittels lebender Mikroorganismen zersetzt und zu Methan umgewandelt werden, zu schaffen, das eine höhere Ausbeute an Methan ergibt sowie geeignet ist, die weiteren Nachteile des Standes der Technik zu vermeiden oder zu verringern.

Diese Aufgabe wird erfindungsgemäß durch ein Verfahren mit den Merkmalen des beigefügten Anspruchs 1 und durch eine Vorrichtung mit den Merkmalen des beigefügten Anspruchs 13 gelöst.

Bevorzugte Ausgestaltungen und Weiterbildungen der Erfindung ergeben sich aus den Unteransprüchen und der nachfolgenden Beschreibung mit zugehörigen Zeichnungen.

Ein erfindungsgemäßes Verfahren zum Erzeugen von methanhaltigem Biogas aus organischen Stoffen, in dem die organischen Stoffe mittels lebender Mikroorganismen zersetzt und zu Methan umgewandelt werden, umfaßt also drei Verfahrensschritte.

In einem ersten Verfahrensschritt der aeroben Fermentation wird organischer Stoff unter aeroben Bedingungen mittels Fermentationsmikroorganismen fermentiert. Dabei werden feste und/oder flüssige Rückstände und CO₂-haltiges Abgas gebildet.

In einem zweiten Verfahrensschritt der Verschwelung werden Rückstände aus dem ersten Verfahrensschritt verschwelt, wobei Holzkohle oder holzkohleähnliche Produkte und Holzgas gebildet werden. Die Rückstände aus dem ersten Verfahrensschritt, insbesondere flüssige Rückstände, werden zur Durchführung der Verschwelung vorteilhafterweise getrocknet. Hierfür sind gebräuchliche Trocknungsverfahren geeignet. Der Wassergehalt sollte vorzugsweise unter 20 % betragen. Das beim Trocknen anfallende Brauchwasser kann in den Biomasse-Aufschwämmungsprozeß wieder eingebracht werden.

In einem dritten Verfahrensschritt der thermophilen Methanfermentation wird Holzgas aus dem zweiten Verfahrensschritt unter anaeroben Bedingungen mittels thermophiler Fermentationsmikroorganismen zu Biogas mit hohem Methananteil fermentiert.

Allgemein wird unter Fermentation der Abbau organischer Stoffe durch geeignete Mikroorganismen wie Hefezellen, Bakterien oder Pilze, insbesondere Schimmelpilze verstanden. In dem ersten Verfahrensschritt der aeroben Fermentation findet eine chemische Umwandlung bzw. Zersetzung des organischen Stoffes durch geeignete Fermentatiosmikroorganismen, insbesondere Bakterien statt. Bei der Auswahl geeigneter Organismen sollte man berücksichtigen, daß die Fermentation in einem flüssigen Nährsubstrat vorteilhafterweise durch hohen Kohlenstoff- und Wasserstoffverbrauch, starke Vermehrung der Mikroorganismen selbst und ihrer Stoffwechselprodukte und/oder ein mikrobiell umgewandeltes Substrat (z.B. Anreicherung von Proteinen) sowie die Möglichkeit der Produktion von Sekundärmetaboliten (z.B. Enzymen, pharmazeutischen Wirkstoffen) gekennzeichnet ist. Beispiele geeigneter Mikroorganismen sind Aspergillus niger, Pyroccocus furiosus und Escherischia coli.

In dem ersten Verfahrensschritt wird der Stickstoff vermindert und das Kohlendioxid angereichert. Die Abbauprodukte umfassen im wesentlichen Biokohle, d.h. ein holzkohlenartiges Produkt als festen Rückstand sowie Kohlendioxid im Abgas. Das Fermentationsprodukt Biokohle ist stark ligninhaltig. Durch die Steuerung der Temperatur und die Auswahl der Mikroorganismen können die Fermentation und die Dynamik des Umsetzungsprozesses gesteuert werden. Die Temperatur liegt vorteilhafterweise im Bereich zwischen 30 °C und 50 °C, besonders bevorzugt zwischen 36 °C und 38 °C.

In dem ersten Verfahrensschritt fällt kein oder nahezu kein Methan an, so daß der Betrieb einer solchen Anlage sicher und umweltverträglich ist.

In dem zweiten Verfahrensschritt der Verschwelung werden Rückstände aus dem ersten Verfahrensschritt nach einer optionalen Trocknung verschwelt. Unter Verschwelen versteht man das Erhitzen bzw. langsame Verbrennen unter gesteuerter Luftarmut oder unter Luftabschluß. Dabei werden außer Mono- und Dimeren die höheren Polymere zu Holzgas und Holzkohleprodukten umgewandelt. Das dabei entstehende Holzkohlenprodukt ist ein Endprodukt und kann einer entsprechenden Verwendung zugeführt werden.

Der zweite Verfahrensschritt wird vorzugsweise in einem Holzvergaser durchgefürt, wo insbesondere das Wirbelschichtverfahren Vorteile bietet. Gegenüber einem konventionellen Holzvergasungssystem kann hierbei vorteilhafterweise die Besonderheit gegeben sein, daß anstelle von normaler Umluft das Abgas aus dem ersten Verfahrensschritt zugeführt wird. Das Zuführen von CO₂-haltigem Abgas aus der aeroben Fermentation bei der Verschwelung, insbesondere zu der Verschwelungsphase kann ein vorteilhaftes Merkmal sein, da die bei der aeroben Fermentation gebildete Umluft mit Kohlendioxid angereichert und an Stickstoff reduziert ist, so daß sie in dem Verschwelungsprozeß, insbesondere der Verschwelungsphase, anstelle von Frischluft weiterverwendet werden kann. Aufgrund der Anreichung des Abgases der aeroben Fermentation mit Kohlendioxid ist somit der Verschwelungsprozeß begünstigt.

Das in dem zweiten Verfahrensschritt entstehende Holzgas weist einen hohen Kohlenmonoxid- und Kohlendioxidanteil auf, der in dem dritten Schritt der thermophilen Methanfermentation zu Methan reduziert wird.

In dem dritten Schritt der thermophilen Methanfermentation werden Mono- und Dimere, d.h. Kohlenmonoxid und Kohlendioxid mikrobiell zu Methan reduziert. Bedarfsweise kann auch eine chemische und/oder physikalische Behandlung durchgeführt werden. Die bei der thermophilen Methanfermentation verwendbaren Mikroorganismen sollten folgende Voraussetzungen erfüllen: Hoher CO₂-Verbrauch, hoher H₂-Verbrauch und starke Vermehrung der methanbildenden Mikroben. Beispiele derartiger Mikroorganismen sind Methona baktericum Thermoautotrophicum, Methanogascina und Methanococcus.

Eine zusätzliche vorteilhafte Besonderheit kann darin bestehen, daß bei der Verschwelung auch weitere organische Stoffe, insbesondere ligninhaltige Stoffe zusammen mit festen und/oder flüssigen Rückständen aus der aeroben Fermentation verschwelt werden können.

Nach einem anderen vorteilhaften Merkmal kann vorgesehen sein, daß bei der thermophilen Methanfermentation auch Rückstände aus der aeroben Fermentation (gegebenenfalls nach Trocknung) zusammen mit Holzgas aus der Verschwelung umgewandelt werden. Dabei kann die thermophile Methanfermentation gesteuert werden.

Nach einem anderen vorteilhaften Merkmal, bei dem ebenfalls eine Steuerung der thermophilen Methanfermentation vorgesehen sein kann, wird vorgeschlagen, daß bei der thermophilen Methanfermentation auch andere kohlenmonoxid- und/oder kohlendioxidhaltige Gase zusammen mit Holzgas aus der Verschwelung zu Methan fermentiert werden.

Als Quellen derartiger kohlenmonoxid- und kohlendioxidhaltiger Gase kommen unter anderem folgende Prozesse in Betracht: Verbrennungsvorgänge, beispielsweise in Heizanlagen oder fossilen Kraftwerken, Motorabgase, Gärvorgänge, beispielsweise in der Gärungsindustrie oder in der Hefeherstellung, Verschwelungsprozesse, chemische Produktionsabläufe, natürliche Rottevorgänge, Abluft aus Gebäuden und industriellen Prozessen sowie Brennstoffzellen. Diese Ausführungsform der Erfindung hat somit den besonderen Vorteil, daß es möglich ist, derartige Abfallstoffe zu energiereichen Methan zu reduzieren sowie gleichzeitig die Umweltbelastung an Kohlenmonoxid bzw. Kohlendioxid zu reduzieren.

Nach einem zusätzlichen vorteilhaften Merkmal wird vorgeschlagen, daß die thermophile Methanfermentation mittels thermophiler Fermentationsmikroorganismen durchgeführt wird, deren Lebensoptimum im Bereich zwischen 18 °C und 90 °C, bevorzugt zwischen 35 °C und 85 °C, besonders bevorzugt zwischen 45 °C oder 55 °C und 65 °C liegt. Erforderlichenfalls ist eine geeignete Beheizungseinrichtung vorzusehen, um die Temperatur der Substanz zu steuern oder zu regeln.

Eine erfindungsgemäße Vorrichtung zum Erzeugen von methanhaltigen Biogas aus organischen Stoffen durch Zersetzung und Umwandlung organischer Stoffe mittels lebender Mikroorganismen, insbesondere zur Durchführung eines erfindungsgemäßen Verfahrens, umfaßt eine aeroben Fermentationsreaktor zum Fermentieren organischer Stoffe unter aeroben Bedingungen mittels Fermentationsmikroorganismen, wobei feste und/oder flüssige Rückstände und CO₂-haltiges Abgas gebildet werden, eine Verschwelungseinrichtung zum Verschwelen von Rückständen aus dem aeroben Fermentationsreaktor, wobei ein Holzkohlenprodukt und Holzgas gebildet werden, sowie einen Methanogenese-Fermentationsreaktor zur Durchführung einer thermophilen Methanfermentation, wobei Holzgas aus der Verschwelungseinrichtung unter anaeroben Bedingungen mittels thermophiler Fermentationsmikroorganismen zu methanhaltigem Biogas fermentiert wird.

Eine entsprechende Anlage, beispielsweise zur Erzeugung von Strom aus Biomasse mittels des erfindungsgemäßen Verfahrens, wird vorteilhafterweise hinsichtlich ihrer Größe nach der Menge der zur Verfügung stehenden Biomasse ausgelegt. Je nach Biomassenverfügbarkeit sind beispielsweise Anlagengrößen von 100 kW, 200 kW, 500 kW, 1 MW oder bis ca. 8 MW zweckmäßig. Eine Anlage von ca. 8 MW benötigt ca. 100 000 Tonnen bis 120 000 Tonnen Biomasse pro Jahr und hat einen Platzbedarf von 10 000 qm bis 15 000 qm.

Das erfindungsgemäße Verfahren und die entsprechende Vorrichtung haben den Vorteil, daß ein Biogas mit einem hohen Methananteil von über 60 % oder 70 % erzeugt werden kann. Es ist möglich, das Verfahren so auszubilden oder zu steuern, daß Biogas mit einem Methangehalt von mindestens 80 %, bevorzugt mindestens 85 % und besonders bevorzugt mindestens 90 % in reproduzierbarer Qualität erzeugt wird.

Das Methan steht somit als Energieträger oder als Ausgangsstoff für chemische Synthesen zur Verfügung. Beispielsweise kann das Biogas bzw. das Methan in kleinen Anlagen der Strom-Wärme-Kopplung, in gasbetriebenen Antriebssystemen oder in Blockheizkraftwerken zur Erzeugung von Strom und Wärme sowie in Gasantrieben verwendet werden.

Ein weiterer Vorteil des erfindungsgemäßen Verfahrens ist darin zu sehen, daß es so durchgeführt oder gesteuert werden kann, daß Biogas mit einem H₂S-Anteil von weniger als 2 %, bevorzugt weniger als 1 % und besonders bevorzugt weniger als 0,5 % erzeugt wird. In der Regel sind Schwefelanteile von weniger als 0,1 % oder 0,05 % zu erzielen. Durch die aerobe Fermentation wird Schwefel zu Sulfat oxidiert, so daß das Biogas im Gegensatz zum Stand der Technik nahezu schwefelfrei ist. Das gewonnene Methan ist industriell nutzbar.

Weiterhin ist der Abbaugrad bei einem erfindungsgemäßen Verfahren gegenüber dem Stand der Technik erhöht und kann bis zu 65 % oder mehr, bezogen auf den organischen Ausgangsstoff, betragen. Auch die mikrobiellen Prozesse laufen reproduzierbar und somit weitgehend störungsfrei ab, da durch die erfindungsgemäße Aufteilung des Abbaus organischer Substanzen in aerobe Fermentation und anaerobe Methanogenese die unterschiedlichen Milieuansprüche der Mikroorganismen jeweils gezielt gesteuert und eingehalten werden können. Bei einer erneuten Inbetriebnahme einer erfindungsgemäßen Anlage kann daher ein wirtschaftliches Leistungsniveau spätestens nach acht Tagen erreicht werden. Ferner arbeitet das erfindungsgemäße Verfahren relativ schnell, so daß die Prozeßzeit für die Umwandlung eines organischen Stoffs in Methan im allgemeinen nur ca. 16 bis 36 Stunden beträgt, wobei das Maximum der Methanproduktion bereits nach ca. 12 Stunden erreicht ist.

Die Endprodukte des erfindungsgemäßen Verfahrens bestehen aus vermarktungsfähiger Holzkohle als festem Rückstand, und je nach verwendetem Ausgangsstoff kann sogar eine teilweise Rückführung eines Endproduktes in den Prozeß, d.h. eine Kaskadennutzung der Biomasse durchgeführt werden. In den einzelnen Bearbeitungsstufen fallen ferner wirtschaftlich interessante Zwischenprodukte an, beispielsweise Pektine, Eiweisse, Pflanzendrogen, z.B. Hecogeninacetat oder Acetylsalicylsäure, die entsprechend extrahiert werden können.

Bei dem erfindungsgemäßen Verfahren verbleiben als Reststoff lediglich mineralische Bestandteile in Höhe von nur etwa 3 % bis 8 % der eingesetzten organischen Stoffe; diese Reststoffe werden deponiert.

In weiteren besonderen Ausführungsformen kann das Verfahren zu dem derart ausgestaltet werden, daß in der thermophilen Methanogenese zusätzliches Kohlendioxid zu Methan reduziert wird, so daß die CO₂-Belastung der Umwelt gesenkt wird.

Mit der Erfindung werden somit Ziele erreicht, um die die Fachwelt sich schon lange bemüht hat. Um dabei besonders gute Ergebnisse zu erzielen, werden die vorstehend erläuterten bevorzugten Merkmale sowie die im folgenden anhand eines in der Figur dargestellten Ausführungsbeispiels näher beschriebenen Besonderheiten einzeln oder in Kombination miteinander eingesetzt, wobei sich durch das Zusammenwirken vorteilhafter Merkmale zusätzliche vorteilhafte Wirkungen ergeben können.

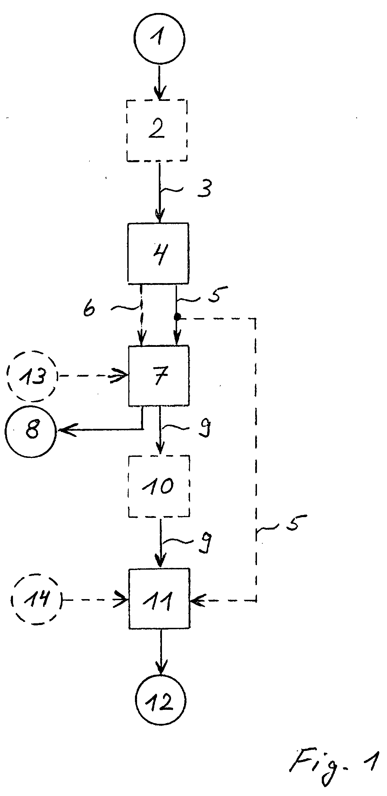

Die Fig. 1 zeigt ein Schema eines erfindungsgemäßen Verfahrens bzw. einer entsprechenden Vorrichtung. Der organische Stoff 1, der als Ausgangsmaterial dient, kann jeder organische Stoff, d.h. ein biologischer organischer Abfallstoff sein, beispielsweise Mist, landwirtschaftliche Abfälle Klärschlämme, Papierschlämme, Küchenabfälle oder Biotonnenrestmüll.

Mit dem erfindungsgemäßen Verfahren verwertbare organische Stoffe 1 sind beispielsweise Brennstoffe, Biomassen und Energiepflanzen. Brennstoffe können beispielsweise Abfälle von Sägewerken, der holzverarbeitenden Industrie oder der Altholzentsorgung, Agrarabfälle, Papier, Papierschlämme und organische Schlämme sein. Geeignete Biomassen sind z.B. alle Arten von organischem Abfall, wie z.B. Nahrungsmittelrückstände, beispielsweise aus der Nahrungsmittelproduktion, Treber, Futtermittelabfälle, verdorbene Lebensmittel, Schlachthofrückstände, Kot, Produktionsrückstände aus der Stärkeherstellung, Küchenabfälle usw..

Als spezifische Beispiele können genannt werden: überlagerte Nahrungsmittel, Spelzen und Getreidestaub, Rückstände aus Konservenfabriken, Melasserückstände, Teigabfälle, schlammförmige Abfälle, überlagerte Genußmittel, Tabakstaub, -gras, -rippen. -schlamm, Zigarettenfehlchargen, Malztreber, Malzkeime, Malzstaub, Hopfentreber, Obst-, Getreide-, und Kartoffelschlempen, Trub und Schlamm aus Brauereien, Schlamm aus Brennereien, Trester, Fabrikationsrückstände von Kaffee oder Tee oder Kakao, Hefe und hefeähnliche Rückstände, Futtermittelabfälle, Ölsaatenrückstände, Fettabfälle (verdorbene Fette), z.B. aus Schlachtung und Margarineherstellung, Inhalte aus Fettabschneidern, Flotate, Molke, Öl-, Fett-, Wachsemulsion, Produktionsrückstände aus der Cremeproduktion, Schlamm aus Speisefett- und Speiseölfabrikation, Bleicherde (entölt), Knochenabfälle und Hautreste, Innereien, Geflügelabfälle, Fischabfälle, Magen-, Darm- und Panseninhalte, Geflügelkot, Schweine- und Rindergülle, Stärkeschlamm, Schlamm aus Gelatinenherstellung, Gelatinestanzabfälle, Rückstände aus Kartoffel-, Mais- und Reisstärkeherstellung, Produktionsrückstände aus der Speiseölfabrikation und von Körperpflegemitteln, Proteinabfälle, Küchen- und Kantinenabfälle (aus Großküchen usw.).

Mögliche verwertbare Energiepflanzen sind z.B. Chinaschilf (Miscantus sinensis giganteus), Weidekulturen, Pappeln, Zuckerhirse und Raps.

Der organische Stoff 1 kann bedarfsweise in einer entsprechenden Vorbereitungsstufe 2 aufbereitet werden, beispielsweise durch Zerkleinern, Trocknen oder Wässern, Formen usw.. Der derart vorbereitete organische Stoff 3 wird dann der ersten erfindungsgemäßen Stufe, einem aeroben Fermentationsreaktor 4 zugeführt, in dem eine chemische Umwandlung/Zersetzung durch FermentationsmikroOrganismen durchgeführt wird, wobei der Stickstoffanteil vermindert und das Kohlendioxid angereichert werden.

Dabei entstehen feste und/oder flüssige Rückstände 5 und kohlendioxidhaltiges Abgas 6. Die aerobe Fermentation wird vorzugsweise derart durchgeführt, daß der organische Stoff dabei in Bewegung gehalten wird, um die Ausbeute zu verbessern und das Verfahren zu beschleunigen. Um eine Zerstörung von Mikroorganismusgesellschaften zu vermeiden, kann dabei vorzugsweise vorgesehen sein, daß der organische Stoff ohne mechanische Hilfswerkzeuge in Bewegung gehalten wird.

Bei der aeroben Fermentation findet ein biochemischer Aufschluß des organischen Stoffs statt, der den Mikroorganismen in der anaeroben Methanogenesestufe eine bessere Möglichkeit der Wirkungsentfaltung bietet. Darüber hinaus kann durch diesen Vorgang Biomasse einer Mehrfachnutzung zugeführt werden. Beispielsweise kann aus Biomasse von Weidenkulturen auf diesem Weg Acetylsalecylsäure gewonnen werden. Ein anderes Beispiel ist die Gewinnung von Pektin aus Produktionsabfällen von Brauereien. Nach einem anderen Beispiel kann aus Abfällen der Sisalagarvenproduktion (Juteherstellung) Hecogeninacetat gewonnen werden. Die verbleibende Restsubstanz wird in dem erfindungsgemäßen Verfahren weiter der Biogasproduktion zugeführt.

Nach einem anderen vorteilhaften Merkmal kann vorgesehen sein, daß der organische Stoff in dem aeroben Fermentationsreaktor 4 mittels eines Luftzufuhr- oder Luftdurchflutungssystems umgewälzt wird. Hierdurch kann sowohl der die für die aerobe Fermentation erforderliche Sauerstoff zugeführt als auch der organische Stoff ohne mechanisches Rührwerk umgewälzt werden.

Die festen und/oder flüssigen Rückstände 5 werden nach einer optionalen Trocknung der nächsten erfindungsgemäßen Stufe, einer Verschwelungseinrichtung 7 zugeführt. Eine solche Verschwelungseinrichtung 7 ist vorzugsweise ein Holzvergaser, der bevorzugt im Wirbelschichtverfahren arbeitet. Nach einem vorteilhaften Merkmal kann vorgesehen sein, daß das kohlendioxidhaltige Abgas 6 auch der Verschwelungseinrichtung 7, insbesondere der Verschwelungsphase zugeführt wird, um den Verschwelungsprozeß zu begünstigen oder die Methanausbeute zu erhöhen.

Die Verschwelungseinrichtung 7 erzeugt als Endprodukt ein Holzkohlenprodukt 8 und ein weiterzuverarbeitendes Holzgas 9 mit einem hohen Kohlenmonoxid und Kohlendioxidanteil, der nach Passieren einer optionalen Gasreinigung 10 der dritten erfindungsgemäßen Stufe, einem Methanogenese-Fermentationsreaktor 11 zugeführt wird. Das Holzgas 9 aus der Verschwelungseinrichtung 7 ist heiß und wird vorzugsweise heiß in den Methanogenese-Fermentationsreaktor 11 eingeleitet. Eine bevorzugte Ausführungsform eines Methanogenese-Fermentationsreaktors 11 ist ein Röhrenreaktor.

In dem Methanogenese-Fermentationsreaktor 11 wird mittels einer thermophilen Methanfermentation das Holzgas 9 unter anaeroben Bedingungen zu Biogas 12 mit einem hohen Methananteil fermentiert. Um dabei der Zerstörung von Mikroorganismusgesellschaften entgegenzuwirken, ist nach einem zusätzlichen vorteilhaften Merkmal vorgesehen, daß der organische Stoff bei der thermophilen Methanfermentation nicht in Bewegung gehalten wird, beispielsweise mittels eines mechanischen Hilfswerkzeugs.

In Fig. 1 ist ferner dargestellt, wie nach einem zusätzlichen vorteilhaften Merkmal vorgesehen sein kann, daß bei der Verschwelung in dem Verschwelungsreaktor 7 auch weitere organische Stoffe 13, insbesondere ligninhaltige Stoffe zusammen mit Rückständen 5 aus dem aeroben Fermentationsreaktor 4 verschwelt werden können.

Ferner ist dargestellt, wie nach einem anderen zusätzlichen Merkmal vorgesehen sein kann, daß dem Methanogenese-Fermentationsreaktor 11 auch Rückstände 5 (gegebenenfalls nach Trocknung) aus der aeroben Fermentation 4 zusammen mit Holzgas 9 aus der Verschwelung zur Umwandlung in Methan zugeführt werden.

Ein besonders vorteilhaftes, optionales Merkmal ist dadurch gegeben, daß bei der thermophilen Methanfermentation 11 auch andere kohlenmonoxid und/oder kohlendioxidhaltige Gase 14, die aus anderen Quellen stammen können, zusammen mit Holzgas 9 aus der Verschwelung 7 zu Methan fermentiert werden können.

### Bezugszeichenliste

- 1: organischer Stoff
- 2: Vorbereitungsstufe
- 3: vorbereiteter organischer Stoff
- 4: aerober Fermentationsreaktor
- 5: Rückstände(fest und/oder flüssig)
- 6: kohlendioxidhaltiges Abgas
- 7: Verschwelungseinrichtung
- 8: Holzkohlenprodukt
- 9: Holzgas
- 10: Gasreinigung
- 11: Methanogenese-Fermentationsreaktor
- 12: Biogas
- 13: weitere organische Stoffe
- 14: andere Gase

## Patentansprüche

1. Verfahren zum Erzeugen von methanhaltigem Biogas (12) aus organischen Stoffen (1), in dem die organischen Stoffe (1) mittels lebender Mikroorganismen zersetzt und zu Methan umgewandelt werden,
**dadurch gekennzeichnet, daß**
in einem ersten Verfahrensschritt der aeroben Fermentation (4) organischer Stoff (1) unter aeroben Bedingungen mittels Fermentationsmikroorganismen fermentiert wird, wobei feste und/oder flüssige Rückstände (5) und CO₂-haltiges Abgas (6) gebildet werden,
in einem zweiten Verfahrensschritt der Verschwelung (7) Rückstände (5) aus dem ersten Verfahrensschritt verschwelt werden, wobei ein Holzkohlenprodukt (8) und Holzgas (9) gebildet werden, und
in einem dritten Verfahrensschritt der thermophilen Methanfermentation (11) Holzgas (9) aus dem zweiten Verfahrensschritt unter anaeroben Bedingungen mittels thermophiler Fermentationsmikroorganismen zu methanhaltigem Biogas (12) fermentiert wird.

2. Verfahren nach Anspruch 1, **dadurch gekennzeichnet, daß** der organische Stoff (1) bei der aeroben Fermentation (4) in Bewegung gehalten wird.

3. Verfahren nach Anspruch 2, **dadurch gekennzeichnet, daß** der organische Stoff ohne mechanische Hilfswerkzeuge in Bewegung gehalten wird.

4. Verfahren nach Anspruch 2 oder 3, **dadurch gekennzeichnet, daß** der organische Stoff (1) mittels eines Luftzufuhr- oder Luftdurchflutungssystems in Bewegung gehalten wird.

5. Verfahren nach einem der vorhergehenden Ansprüche, **dadurch gekennzeichnet, daß** bei der Verschwelung (7) auch weitere organische Stoffe (13), insbesondere ligninhaltige Stoffe zusammen mit Rückständen (5) aus der aeroben Fermentation (4) verschwelt werden.

6. Verfahren nach einem der vorhergehenden Ansprüche, **dadurch gekennzeichnet, daß** bei der Verschwelung (7) CO₂-haltiges Abgas (6) aus der aeroben Fermentation (4) zugeführt wird.

7. Verfahren nach einem der vorhergehenden Ansprüche, **dadurch gekennzeichnet, daß** bei der thermophilen Methanfermentation (11) auch Rückstände (5) aus der aeroben Fermentation (4) zusammen mit Holzgas (9) aus der Verschwelung (7) umgewandelt werden.

8. Verfahren nach einem der vorhergehenden Ansprüche, **dadurch gekennzeichnet, daß** bei der thermophilen Methanfermentation (11) auch andere CO- und/oder CO₂-haltige Gase (14) zusammen mit Holzgas (9) aus der Verschwelung (7) zu Methan fermentiert werden.

9. Verfahren nach einem der vorhergehenden Ansprüche, **dadurch gekennzeichnet, daß** der organische Stoff (1) bei der thermophilen Methanfermentation (11) nicht in Bewegung gehalten wird.

10. Verfahren nach einem der vorhergehenden Ansprüche, **dadurch gekennzeichnet, daß** die thermophile Methanfermentation (11) mittels thermophiler Fermentationsmikroorganismen durchgeführt wird, deren Lebensoptimum im Bereich zwischen 18 °C und 90 °C, bevorzugt zwischen 35 °C und 85 °C, besonders bevorzugt zwischen 45 °C oder 55 °C und 65 °C liegt.

11. Verfahren nach einem der vorhergehenden Ansprüche, **dadurch gekennzeichnet, daß** es so gesteuert wird, daß Biogas (12) mit einem Methangehalt von mindestens 80 %, bevorzugt mindestens 85 %, besonders bevorzugt mindestens 90 % erzeugt wird.

12. Verfahren nach einem der vorhergehenden Ansprüche, **dadurch gekennzeichnet, daß** es so gesteuert,wird, daß Biogas (12) mit einem H₂S-Anteil von weniger als 2 %, bevorzugt weniger als 1 %, besonders bevorzugt weniger als 0,5 %, 0,1 % oder 0,05 % erzeugt wird.

13. Vorrichtung zum Erzeugen von methanhaltigem Biogas (12) aus organischen Stoffen (1) durch Zersetzung und Umwandlung organischer Stoffe (1) mittels lebender Mikroorganismen, insbesondere zur Durchführung eines Verfahrens nach einem der Ansprüche 1 bis 12,
umfassend
einen aeroben Fermentationsreaktor (4) zum Fermentieren organischer Stoffe (1) unter aeroben Bedingungen mittels Fermentationsmikroorganismen, wobei feste und/oder flüssige Rückstände (5) und CO₂- haltiges Abgas (6) gebildet werden,
eine Verschwelungseinrichtung (7) zum Verschwelen von Rückständen (5) aus dem aeroben Fermentationsreaktor (4), wobei ein Holzkohlenprodukt (8) und Holzgas (9) gebildet werden, und
einen Methanogenese-Fermentationsreaktor (11) zur Durchführung einer thermophilen Methanfermentation, wobei Holzgas (9) aus der Verschwelungseinrichtung (7) unter anaeroben Bedingungen mittels thermophiler Fermentationsmikroorganismen zu methanhaltigem Biogas (12) fermentiert wird.

14. Vorrichtung nach Anspruch 13, **dadurch gekennzeichnet, daß** der aerobe Fermentationsreaktor (4) eine Einrichtung zum in Bewegung halten der organischen Stoffe (1) aufweist.

15. Vorrichtung nach Anspruch 14, **dadurch gekennzeichnet, daß** der aerobe Fermentationsreaktor (4) kein mechanisches Hilfswerkzeug zum in Bewegung halten der organischen Stoffe (1) aufweist.

16. Vorrichtung nach Anspruch 14 oder 15, **dadurch gekennzeichnet, daß** der aerobe Fermentationsreaktor (4) ein Luftzufuhr- oder Luftdurchflutungssystem zum in Bewegung halten der organischen Stoffe (1) aufweist.

17. Vorrichtung nach Anspruch 13, **dadurch gekennzeichnet, daß** der Methanogenese-Fermentationsreaktor (11) keine Einrichtung zum in Bewegung halten der organischen Stoffe (1) aufweist.
